# FASCICULE DE BREVET EUROPEEN

(11) **EP 3 282 836 B1**
(45) Date de publication et mention de la délivrance du brevet: **04.12.2019**
(21) Numéro de dépôt: 16722285.0
(22) Date de dépôt: 12.04.2016
(51) Int. Cl.: A01K 67/033

(54) **PROCEDE D'ELEVAGE D'INSECTES**
VERFAHREN ZUR INSEKTENZÜCHTUNG
METHOD FOR INSECT BREEDING

(30) Priorité: 13.04.2015 FR 1553208
(43) Date de publication de la demande: 21.02.2018
(73) Titulaire: Ynsect, 91058 Evry Cedex (FR)
(72) Inventeur: COMPARAT, Solène, 91000 Evry (FR); HUBERT, Antoine, 94140 Alfortville (FR); BERRO, Fabrice, 75001 Paris (FR); LEVON, Jean-Gabriel, 75011 Paris (FR)
(74) Mandataire: Santarelli
(86) Numéro de dépôt international: PCT/FR2016/050843
(87) Numéro de publication internationale: WO 2016/166465

(56) Documents cités:
- WO-A1-2014/171829

## Description

La présente invention concerne le domaine de l'élevage d'insectes. En particulier, elle porte sur un procédé d'élevage d'insectes.

Les insectes, en particulier certaines espèces, peuvent constituer une source de produits ou de matières premières, notamment pour l'alimentation animale ou humaine, ou à destination de nombreuses autres industries.

Sauf indication contraire, le terme « insecte » employé dans le présent document désigne tout stade d'évolution de l'œuf ou oothèque à l'insecte adulte, en passant par la larve et la nymphe ou pupe.

En particulier, le terme « larve » désigne dans le présent document le stade larvaire des insectes, ce qui inclut l'asticot pour les diptères et la chenille pour les lépidoptères, ainsi que les stades aptères chez les orthoptères. Le terme « nymphe » désigne dans le présent les stades intermédiaires entre la larve et l'imago, ce qui inclut la pupe pour les diptères, la nymphe pour les coléoptères, la chrysalide pour les lépidoptères et, le cas échéant, un stade intermédiaire au cours duquel certaines modifications physiologiques (prépupe) ou comportementales des individus apparaissent, telles qu'une sclérification importante de la cuticule pour les diptères. De la même manière, le terme « œuf » couvre également une oothèque de dictyoptères.

Typiquement, certaines espèces d'insectes comestibles sont riches en protéines. Environ deux mille espèces d'insectes comestibles ont été identifiées à ce jour, et ce nombre croît régulièrement. De nombreux insectes peuvent être employés pour l'alimentation des animaux d'élevage terrestres (mammifères, oiseaux...), des poissons et invertébrés aquatiques d'élevage, etc. Les insectes convertissent de manière générale une grande proportion de ce qu'ils ingèrent en masse corporelle (notamment nettement plus que les mammifères). En effet, ils ont un métabolisme d'organismes poïkilothermes qui ne nécessite pas d'utiliser de l'énergie pour maintenir leur température corporelle. A contrario, les animaux supérieurs, dits homéothermes, emploient une énergie importante pour maintenir leur température corporelle. La domestication des insectes à des fins de production alimentaire constitue ainsi une opportunité vis-à-vis des enjeux mondiaux en matière de nutrition et de préservation de l'environnement.

Outre l'aspect alimentaire, les insectes peuvent constituer une ressource importante dans de nombreux domaines industriels. Typiquement, l'exosquelette des insectes est constitué en grande partie de chitine, dont un dérivé connu est le chitosan. Les applications de la chitine et/ou du chitosan sont nombreuses: cosmétique (composition cosmétique), médicale et pharmaceutique (composition pharmaceutique, traitement des brûlures, biomatériaux, pansements cornéens, fils chirurgicaux), diététique et alimentaire, technique (agent filtrant, texturant, floculant ou adsorbant notamment pour la filtration et dépollution de l'eau), etc. En effet, la chitine et /ou le chitosan sont des matériaux biocompatibles, biodégradables et non toxiques.

L'élevage des insectes connaît ainsi un certain essor. Certaines méthodes et certains dispositifs relatifs à un tel élevage ont ainsi été développés. On connaît par exemple au travers du document WO2014/171829 un procédé et un dispositif associé permettant d'automatiser l'apport en nourriture dans des caisses d'élevage d'insectes. Plus précisément, ce document divulgue un dispositif permettant de déterminer, par un procédé d'observation de chacune des caisses d'un élevage, l'état et le stade de croissance des insectes présents dans chacune des caisses, et si un apport de nourriture est nécessaire dans la caisse considérée.

Ainsi, si certains procédés connus répondent à certaines problématiques de simplification dans un élevage d'insectes, il n'est pas connu de procédé particulièrement adapté à l'élevage d'insectes à grande échelle.

En particulier, l'optimisation de la productivité d'un élevage à grande échelle reste une problématique inconnue ou résolue de manière insatisfaisante dans l'état de la technique. Or, un élevage à grande échelle permettrait l'obtention de quantités de produits suffisantes pour intéresser les marchés de commodités alimentaires et chimiques notamment.

La présente invention a pour but de résoudre au moins l'un des inconvénients précités. La présente invention vise notamment à proposer un dispositif, en particulier un atelier, optimisant la logistique associée à l'élevage d'insectes et de manière générale la conduite de l'élevage sur un cycle complet de production d'insectes.

En particulier, l'invention porte sur un procédé d'élevage d'insectes comportant des phases de croissance lors desquelles les insectes sont stockés dans un environnement contrôlé, lesdites phases de croissance alternant avec des séquences opératoires lors desquelles au moins une opération ponctuelle d'élevage est réalisée. L'opération ponctuelle d'élevage est l'une des opérations suivantes :
- déposer un substrat d'élevage dans un contenant d'élevage ;
- apporter de l'eau dans un contenant d'élevage ;
- vider un contenant d'élevage ;
- identifier des insectes présentant des symptômes de maladie pour les séparer d'un contenant ;
- séparer des déchets fins, comprenant des déjections, des restes de substrat et des mues, et les insectes ;
- séparer des larves vivantes matures et le substrat non consommé ;
- séparer des larves mortes de larves vivantes ;
- calibrer des larves selon leur taille ;
- trier les insectes adultes des larves et des nymphes ;
- trier les insectes vivants des insectes morts ;
- trier des œufs des adultes et trier des œufs du substrat ;
- trier des nymphes des larves ;
- nettoyer un contenant d'élevage ;
- remplir un contenant d'élevage avec des insectes ;
- jeter le contenu d'un contenant d'élevage,

Le procédé objet de l'invention comprend une séquence dite de synchronisation lors de laquelle un lot d'insectes est trié et réparti en plusieurs catégories de taille ou de maturité dans différents contenants, puis lesdits contenants sont regroupés pour constituer des unités élémentaires d'élevage comprenant un nombre prédéfini de contenants, une unité élémentaire comprenant uniquement des insectes de même catégorie.

Un tel procédé, rendu possible par l'échelle importante de l'élevage dans lequel il est appliqué, permettant la constitution de lots significatifs d'insectes de même stade de développement ou de même maturité, permet de séquencer les opérations d'élevage à appliquer de manière synchrone sur une unité élémentaire d'élevage ou plusieurs unités élémentaires d'élevage.

Les séquences d'élevages, c'est-à-dire les ensembles d'opérations à effectuer, peuvent être prédéterminées et réalisées sans suivi autre que temporel de l'élevage, ou en s'appuyant sur de simples contrôles ponctuels de la croissance des insectes à des moments prédéterminés.

Le procédé s'en trouve optimisé et facilement automatisable, comparativement par exemple à un élevage artisanal en bacs individuels dans lequel chaque bac doit être géré individuellement et trié. Le cas échéant, le groupement des contenants en unités élémentaires (typiquement palettisées), permet une gestion industrielle optimisée de l'élevage à différents niveaux : par contenant, par unité élémentaire d'élevage, par lots d'unités élémentaires.

Les opérations d'élevage et de contrôle peuvent être réalisées de manière automatisée, et leur réalisation rationnalisée. Le fait d'automatiser les opérations permet d'augmenter la robustesse du procédé et la qualité du produit final et d'éviter toute erreur humaine. Cela permet également de collecter plus de données relatives à l'élevage, et ce plus rapidement, grâce à des capteurs (pouvant notamment opérer en continu) et à l'automatisation de tout ou partie des opérations.

La récolte du ou des produits finaux de l'élevage (insectes adultes de maturité donnée, larves, etc.) est réalisée par unité élémentaire. Du fait de la synchronisation des insectes au cours de l'élevage, les insectes récoltés présentent dans chaque unité élémentaire une bonne homogénéité. En particulier, lors de la récolte des insectes en vue de leur envoi vers un procédé de valorisation, le taux d'éléments indésirables (par exemple d'insectes n'étant pas au calibre souhaité, d'insectes morts, de déjections) est faible.

Ainsi, la formation d'unités élémentaires d'élevage, comportant des insectes au même stade d'évolution, permet une gestion séquentielle simple du procédé d'élevage mis en œuvre dans l'atelier. Ainsi, une gestion de production de type industrielle jusqu'alors inconnue dans le domaine de l'élevage des insectes peut être mise en œuvre avec typiquement une automatisation complète de l'ensemble des opérations de manutention, de nourrissage, de contrôle et d'observation des unités élémentaires.

Selon une variante de l'invention, la séquence de synchronisation peut être menée plusieurs fois sur chaque insecte au cours dudit procédé.

Selon un mode de réalisation, le procédé peut comprendre une séquence dite de concentration/dilution des insectes, dans laquelle la densité d'insectes dans chaque contenant d'une unité d'élevage est amenée à une densité cible par ajout d'insectes, retrait d'insectes, ou répartition des insectes dans des contenants, la densité cible correspondant à une densité prédéterminée souhaitée au début de la phase de stockage subséquente.

La densité cible peut correspondre à la densité pour laquelle il est estimé que, tenant compte de la croissance des insectes prévue lors de la phase de stockage subséquente, l'intervalle temporel souhaité séparant la séquence de concentration/dilution courante d'une séquence de concentration/dilution suivante s'achèvera avec une densité considérée maximale garantissant la bonne santé des insectes dans les contenants.

La densité cible peut alternativement être définie par un ratio de la densité dans les contenants avant la séquence de concentration/dilution.

La densité d'insectes peut notamment être soit :
- une masse d'insectes par volume ou par surface de contenant ; soit
- un nombre d'insectes par volume ou par surface de contenant.

Selon un mode de réalisation, le procédé peut combiner :
- un procédé dit de production, pour l'élevage d'insectes, de l'œuf jusqu'au stade de larve ayant une maturité prédéfinie ; ou
- un procédé dit de reproduction, pour l'élevage d'insectes de l'œuf ou du stade de juvénile jusqu'au stade de jeune adulte, associé à un procédé dit de ponte, portant sur la production d'œufs par des insectes adultes.

Le procédé peut comporter, entre chaque phase de croissance, une étape de convoyage automatisé des unités élémentaires d'élevage contenant les insectes devant faire l'objet d'au moins une séquence opératoire, depuis une zone de stockage dans laquelle se déroule ladite phase de croissance, vers une zone opératoire comportant les postes de travail adaptés des opérations d'élevage de la séquence opératoire.

Le procédé peut comporter des séquences opératoires de nourrissage.

Le procédé peut comporter des séquences opératoires d'apport d'eau.

Le procédé peut comporter une séquence opératoire de prélèvement d'insectes adultes pour les destiner à la ponte.

Le procédé peut comporter les séquences opératoires suivantes :
- la récupération des œufs des contenants d'élevage contenant des insectes destinés à la ponte,
- la concentration des œufs dans des contenants d'élevage.

Le procédé peut comporter les séquences opératoires suivantes :
- la récupération des juvéniles des contenants (31, 32) d'élevage contenant des insectes destinés à la ponte,
- la concentration des juvéniles dans des contenants (31, 32) d'élevage.

Le procédé peut comporter une séquence opératoire de vidage et nettoyage des contenants d'élevage en vue de leur réemploi.

Les séquences opératoires peuvent comporter la succession ordonnée de plusieurs opérations parmi les opérations suivantes :
- Stocker une unité élémentaire d'élevage en environnement contrôlé ;
- Déstocker une unité élémentaire d'élevage ;
- Dégrouper partiellement ou totalement les contenants d'élevage d'une unité élémentaire d'élevage ;
- Grouper en unité élémentaire d'élevage des contenants d'élevage ;
- Déposer du substrat d'élevage dans un contenant d'élevage ;
- Apporter de l'eau dans un contenant d'élevage ;
- Vider un contenant d'élevage ;
- Identifier les individus présentant des symptômes de maladie pour les séparer du contenant ;
- Séparer les déchets fins, comprenant les déjections, les restes de substrat et les mues, des insectes ;
- Séparer les larves vivantes matures et le substrat non consommé ;
- Séparer les larves mortes des larves vivantes ;
- Calibrer des larves selon leur taille ;
- Trier les insectes adultes des larves et des nymphes ;
- Trier les insectes vivants des insectes morts ;
- Trier les œufs des adultes et trier les œufs du substrat ;
- Trier les nymphes des larves ;
- Nettoyer un contenant d'élevage ;
- Remplir un contenant d'élevage avec des insectes ;
- Sortir une unité d'élevage de l'élevage et l'envoyer vers un autre procédé ;
- Faire entrer une nouvelle unité d'élevage ;
- Jeter le contenu d'un contenant d'élevage.

En particulier, la séquence de synchronisation peut être réalisée au stade larvaire par mise en œuvre d'une séquence de calibration des larves comportant les opérations suivantes :
- Déstocker une unité élémentaire d'élevage ;
- Dégrouper partiellement ou totalement les contenants d'élevage d'une unité élémentaire d'élevage ;
- Vider les contenants d'élevage ;
- Nettoyer les contenants d'élevage ;
- Séparer les déjections et les larves vivantes ;
- Calibrer des larves selon leur taille ;
- Remplir des contenants d'élevage avec les larves vivantes ;
- Déposer du substrat d'élevage dans les contenants d'élevage ;
- Grouper en unité élémentaire d'élevage les contenants d'élevage ;
- Stocker les unités élémentaires d'élevage en environnement contrôlé.

En particulier, la séquence de concentration/dilution peut comprendre les opérations suivantes :
- Déstocker une unité élémentaire d'élevage ;
- Dégrouper totalement les contenants d'élevage de l'unité élémentaire d'élevage ;
- Vider les contenants d'élevage ;
- Nettoyer les contenants ;
- Séparer les déjections et les larves vivantes ;
- Remplir des contenants d'élevage avec des insectes, à la densité souhaitée ;
- Déposer du substrat d'élevage;
- Grouper en unités élémentaires d'élevage les contenants d'élevage ;
- Stocker les unités élémentaires d'élevage en environnement contrôlé.

La séquence de synchronisation et la séquence de concentration/dilution peut comporter en outre une opération d'apport d'eau.

D'autres particularités et avantages de l'invention apparaîtront encore dans la description ci-après.

Aux dessins annexés, donnés à titre d'exemples non limitatifs :
- la figure 1 présente, selon une vue schématique en trois dimensions, un exemple d'atelier permettant la mise en œuvre d'un procédé conforme à un mode de réalisation de l'invention ;
- La figure 2 présente, selon une vue schématique, une unité élémentaire pour l'élevage d'insectes ;
- La figure 3 présente, selon une vue schématique, un exemple d'organisation d'une seconde zone d'un atelier permettant la mise en œuvre d'un procédé conforme à un mode de réalisation de l'invention.
- La figure 4 présente, sous forme d'un logigramme, une séquence opératoire particulière de calibration de larves, pouvant être mise en œuvre pour la synchronisation des insectes ;
- La figure 5 présente, sous forme d'un logigramme, une séquence opératoire particulière dite de concentration/dilution ;
- La figure 6 représente schématiquement, trois procédés pouvant coexister dans un élevage d'insectes, dans l'exemple particulier de l'élevage de ténébrions meuniers.

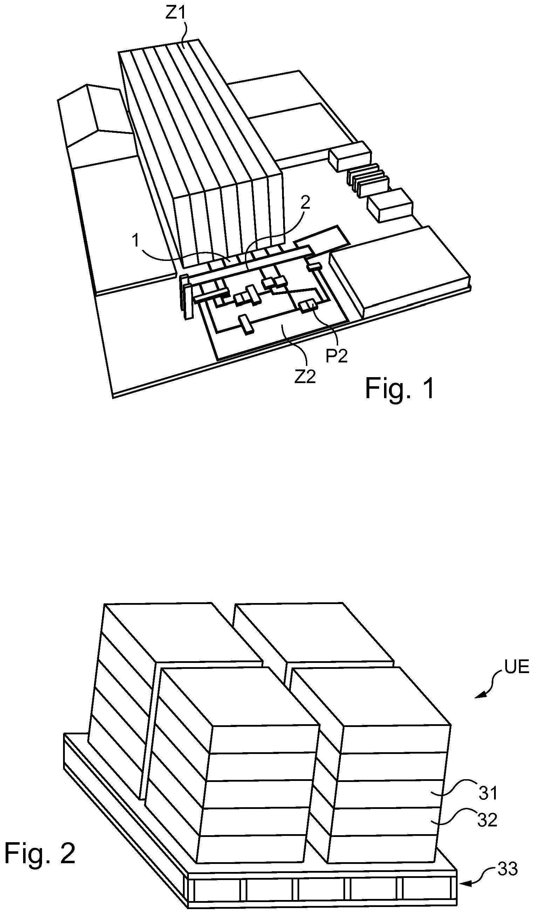

La figure 1 représente un atelier d'élevage d'insectes, ici représenté sous la forme d'une vue schématique en trois dimensions, particulièrement adapté à la mise en œuvre d'une variante préférentielle du procédé objet de l'invention.

L'atelier représenté comporte au moins deux zones, à savoir une première zone Z1 organisée pour le stockage des insectes pendant leur croissance, c'est-à-dire pendant les phases de croissance du procédé objet de l'invention. Dans cette première zone Z1, les insectes grandissent dans des conditions environnementales (définies par des paramètres environnementaux dont la température, l'hygrométrie...) contrôlées, dirigées et optimisées.

Tel que précédemment mentionné, la notion d'élevage d'insectes comprend la croissance d'insectes adultes jusqu'à un stade désiré, mais peut également comprendre toutes les phases précédant l'obtention d'un insecte adulte (ou imago), depuis la ponte des œufs (ou oothèque) en passant par leur éclosion, le stade larvaire, l'éventuel stade de nymphe, de pupe (l'ensemble des stades intermédiaires), etc.

Dans une variante du procédé objet de l'invention, l'élevage d'insectes peut notamment être envisagé comme un ensemble organisé permettant la ponte d'œufs par des insectes adultes pour la production de larves, certaines larves étant élevées jusqu'au stade adulte pour la ponte de nouveaux œufs, les adultes étant renouvelés régulièrement (par exemple suite à leur mort) par des adultes jeunes assurant de nouvelles pontes et ainsi de suite. Le produit final de la production peut être des œufs, et/ou des larves, et/ou des nymphes, et/ou des insectes adultes.

L'atelier ici représenté comporte également une seconde zone Z2, organisée pour la réalisation d'une ou plusieurs séquences ou opérations d'élevage. La conduite de l'élevage comporte la mise en œuvre d'une succession de séquences ou d'opérations d'élevage. Une séquence ou « séquence opératoire » comporte une ou plusieurs opérations successives prédéfinies, et est réalisée entre deux phases de croissance (sauf lorsqu'il s'agit d'envoyer les insectes vers un autre procédé).

Les opérations d'élevage correspondent à des opérations devant être menées pour le maintien en vie, la bonne croissance et/ou l'optimisation des conditions d'élevage des insectes.

La seconde zone Z2 comporte en particulier un ou plusieurs postes P1, P2 de travail spécialisés dans la réalisation d'une ou plusieurs opérations d'élevage. En particulier, la seconde zone Z2 peut être conformée pour permettre la mise en œuvre, à un poste ou plusieurs postes de travail, de séquences d'élevage constituées d'une succession d'opérations unitaires. Les postes de travail peuvent typiquement être regroupés en ilots pour la mise en œuvre d'opérations successives. Les postes P1, P2, ou ilots de postes peuvent être desservis par un convoyeur à bande 2.

Les insectes (œufs, larves, nymphes, ou adultes) sont élevés dans des contenants groupés en unités élémentaires d'élevage, préférentiellement sous la forme de palettes. Lors de phases de croissance, les palettes sont stockées dans la première zone Z1, par exemple dans des rayonnages à palettes.

Afin de mener des opérations d'élevage identiques sur tous les contenants et tous les insectes d'une même unité élémentaire d'élevage, une séquence dite de synchronisation peut être mise en œuvre dans le procédé, séquence lors de laquelle un lot d'insectes est trié de sorte à répartir les insectes (œufs, larves, nymphes, pupes, adultes...) par taille ou maturité dans différents contenants, puis à regrouper les contenants pour constituer des unités élémentaires d'élevage comprenant un nombre prédéterminé de contenants. En particulier, les contenants sont regroupés de sorte qu'une même unité élémentaire contienne des insectes sensiblement au même stade de développement ou de même maturité. Dans une unité élémentaire constituée pendant la séquence de synchronisation, selon la précision du tri ou de calibration effectuée, typiquement au moins 80% d'insectes présentent sensiblement la même maturité, et de manière préférentielle au moins 90% d'insectes présentent sensiblement la même maturité.

La synchronisation peut typiquement être réalisée peu après la ponte d'œufs ou oothèque, de sorte qu'une unité élémentaire d'élevage ne contienne, au début d'un cycle d'élevage, que des œufs pondus au plus à quelques jours d'intervalle. Une séquence de synchronisation peut être prévue plusieurs fois entre la ponte d'un œuf et l'obtention du produit final de l'élevage, et/ou entre la ponte d'un œuf et l'obtention d'un insecte adulte. Typiquement, la synchronisation peut être réalisée en mettant en œuvre une séquence de calibration, par exemple par taille, au stade larvaire.

Pour la réalisation de certaines opérations, notamment des opérations de tri ou calibration pendant une séquence de synchronisation, il peut s'avérer nécessaire de dé-palettiser et/ou dégrouper les contenants d'élevage. Cette opération peut, selon diverses organisations possibles, être réalisée au niveau d'une interface 1, ou sur un poste dédié de la seconde zone Z2.

Dans l'invention, la croissance des insectes, c'est-à-dire les phases d'élevage en dehors des opérations ponctuelles d'élevage, s'effectue dans des unités élémentaires d'élevage ayant fait l'objet d'une séquence de synchronisation. A simple titre d'exemple, la figure 2 illustre un exemple d'unité élémentaire UE d'élevage, selon une représentation de principe en trois dimensions. En particulier, les contenants 31, 32 d'élevage peuvent être des caisses ou bacs empilables. Par bacs ou caisses empilables, on désigne en particulier des bacs ou caisses qui se superposent les unes au-dessus des autres, de manière légèrement encastrée, ce qui procure une certaine stabilité à la colonne de caisses ainsi formée.

Tel que représenté à la figure 2, les contenants 31, 32 sont palettisés, c'est-à-dire groupés en unités élémentaires UE sur une palette 33 de manutention. La palette 33 peut notamment, mais pas exclusivement, être une palette de taille classique, c'est-à-dire typiquement une palette de type « palette Europe », ou une demi-palette de ce type.

A titre d'exemple, une unité élémentaire UE d'élevage peut typiquement regrouper de huit à cent contenants, et comporter une, deux, trois, quatre piles de contenants, voire plus. La hauteur d'une unité élémentaire d'élevage complète peut par exemple être comprise entre 160 et 230 cm, et typiquement de l'ordre de 200cm.

Lors des phases dites de croissance, chaque unité élémentaire peut être stockée dans une partie ou silo de la première zone Z1 présentant des conditions environnementales optimisées pour le stade de développement (ou la maturité) des insectes de l'unité élémentaire considérée.

Les silos sont isolés entre eux par un cloisonnement adapté. Ce cloisonnement des silos peut mettre en œuvre des lames d'air, ou tout autre moyen de cloisonnement permettant de séparer deux zones afin de pouvoir y garantir deux conditions atmosphériques (température, hygrométrie, ...) différentes et un confinement sanitaire entre les silos. Par exemple, des cloisonnements physiques peuvent être mis en œuvre. La première zone Z1 peut comporter plusieurs magasins différents, séparés par des cloisons physiques.

Les silos ainsi constitués peuvent être dédiés à différents stades de maturité des insectes, ou à plusieurs procédés d'élevage, conforme à des modes de réalisation de l'invention et menés en parallèle dans un élevage.

Par exemple, la conduite de l'élevage peut comporter plusieurs cycles auxquels peuvent être associés des conditions d'élevages différentes. Typiquement, l'élevage peut comporter :
- un cycle d'incubation pour la production de juvéniles par des adultes fertiles, ce cycle étant mené à une température et dans des conditions d'humidité relativement élevées ;
- un cycle de reproduction, du juvénile au jeune adulte mature fertile en passant par la nymphose dans des conditions environnementales adaptées;
- un cycle de production (ou « d'engraissement ») du juvénile à la larve mature pour l'abattage, avec température et humidité plus basse.

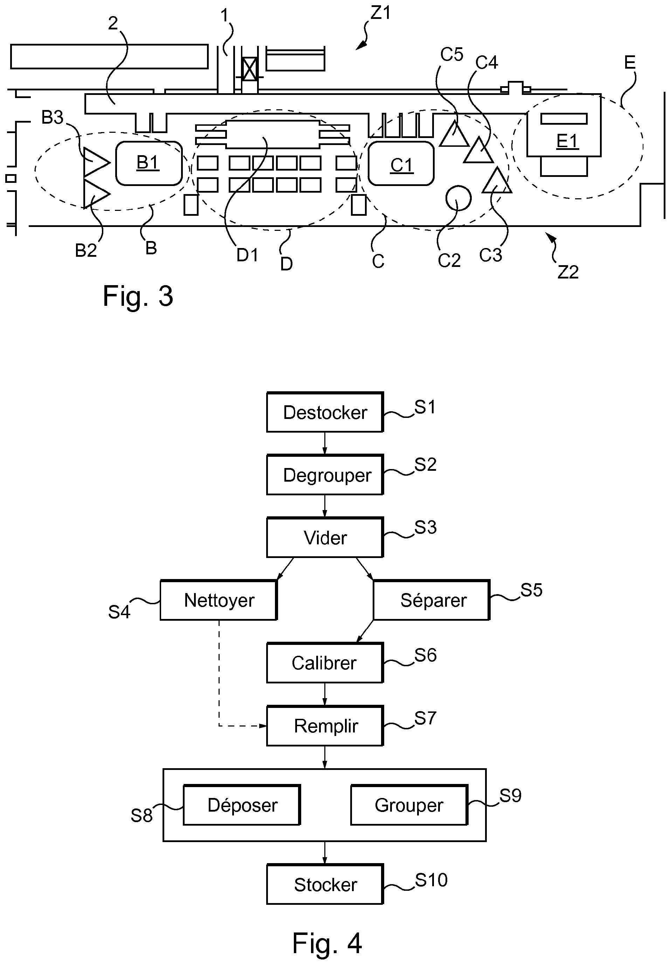

La figure 3 présente, selon une vue schématique, un exemple d'organisation d'une seconde zone Z2 d'un atelier adapté à la mise en œuvre d'un procédé conforme à un mode de réalisation de l'invention.

L'exemple de seconde zone Z2 présenté à la figure 8 est représenté dans le cadre de l'exemple d'atelier de la figure 1. En particulier, on a représenté à la figure 3 l'interface 1 avec la première zone Z1. Un système de convoyage, à savoir dans l'exemple représenté un convoyeur à bande, 2 assure le déplacement des unités élémentaires ou, le cas échéant de contenants dégroupés. Un transstockeur, après sélection d'une palette dans la première zone Z1, dépose celle-ci à l'entrée du système de convoyage, à savoir typiquement sur une zone du convoyeur à bande 2 faisant interface 1 entre la première zone Z1 et la seconde zone Z2, ou tout autre dispositif permettant l'envoi au moment souhaité de la palette sur ledit convoyeur à bande 2. Dans l'exemple ici représenté, les unités élémentaires palettisées sont dirigées par le convoyeur à bande 2 vers des zones de dé-palettisation (et palettisation) en l'occurrence une première zone logistique B1 et une seconde zone logistique B2.

De manière générale, l'exemple de seconde zone Z2 ici représentée est organisée en quatre sous-zones appelées ilots, respectivement référencés B, C, D et E. Les ilots B, C, D et E sont associés à une ou plusieurs opérations d'élevage, pour lesquels ils sont plus ou moins spécialisés.

De manière générale, la réalisation des séquences opératoires mises en œuvre dans l'invention peut comporter l'une ou plusieurs des principales opérations d'élevage suivantes (et, le cas échéant, d'autres opérations complémentaires) :
- Stocker une unité élémentaire d'élevage en environnement contrôlé ;
- Déstocker une unité élémentaire d'élevage ;
- Dégrouper partiellement ou totalement les contenants d'élevage d'une unité élémentaire d'élevage ;
- Grouper en unité élémentaire d'élevage des contenants d'élevage ;
- Déposer du substrat d'élevage dans un contenant d'élevage ;
- Apporter de l'eau dans un contenant d'élevage ;
- Vider un contenant d'élevage ;
- Identifier les individus présentant des symptômes de maladie pour les séparer du contenant ;
- Séparer les déchets fins, comprenant les déjections, les restes de substrat et les mues, des insectes ;
- Séparer les larves vivantes matures et le substrat non consommé ;
- Séparer les larves mortes des larves vivantes ;
- Calibrer des larves selon leur taille ;
- Trier les insectes adultes des larves et des nymphes ;
- Trier les insectes vivants des insectes morts ;
- Trier les œufs des adultes et trier les œufs du substrat ;
- Trier les nymphes des larves ;
- Nettoyer un contenant d'élevage ;
- Remplir un contenant d'élevage avec des insectes ;
- Sortir une unité d'élevage de l'élevage et l'envoyer vers un autre procédé ;
- Faire entrer une nouvelle unité d'élevage ;
- Jeter le contenu d'un contenant d'élevage.

Le substrat d'élevage correspond au milieu ajouté dans les contenants, et qui est favorable à la vie et au développement des insectes ou larves ou nymphes. Le substrat employé peut être sous la forme d'un solide sec, d'un solide humide, ou être liquide.

Dans l'exemple représenté, l'ilot E correspond à un poste adapté à la réalisation d'une séquence de nourrissage des insectes (ou larves, ou nymphes, etc.). Un dispositif de nourrissage E1 équipe l'ilot de nourrissage E.

Selon diverses variantes de l'invention, le nourrissage nécessite ou non la dé-palettisation et le dégroupement des contenants formant les unités élémentaires d'élevage, c'est-à-dire l'opération ci-avant désignée « dégrouper partiellement ou totalement les contenants d'élevage d'une unité élémentaire d'élevage ». Ainsi, la dé-palettisation peut consister à séparer chaque contenant d'une unité élémentaire les uns des autres, afin d'obtenir un ensemble de contenants individuels, ou à séparer une unité élémentaire en groupes de contenants (de typiquement quatre à six contenants).

La dé-palettisation et la palettisation, dans l'ilot de nourrissage E tout comme au niveau des première et deuxième zones logistiques B1, C1, peut par exemple être réalisée à l'aide d'un robot de manutention poly-articulé, par exemple un robot six-axes, ou un robot sept axes. Un tel robot peut permettre de manière plus générale la manipulation des contenants d'élevage avec des vitesses, des accélérations, et un maintien en position compatible de l'élevage d'insectes.

Le dispositif de nourrissage E1, que les contenants de l'unité élémentaire soient ou non dégroupés, assure une répartition sensiblement uniforme de la nourriture dans les contenants.

L'ilot de nourrissage E peut optionnellement permettre un apport en eau dans les contenants d'élevage. Cet apport en eau est réalisable selon diverses modalités, alternatives ou complémentaires : par remplissage périodique d'un réservoir dédié des contenants, par brumisation, par apport d'une nourriture riche en eau ou enrichie en eau.

Ainsi, une séquence de nourrissage peut comprendre la séquence des opérations suivantes :
- Déstocker une unité élémentaire d'élevage (de la première zone Z1) ;
- Dégrouper partiellement ou totalement les contenants d'élevage d'une unité élémentaire d'élevage (opération optionnelle selon la variante de l'invention considérée);
- Séparer les déjections des contenants (opération optionnelle dans cette séquence) ;
- Déposer du substrat d'élevage dans les contenants d'élevage ;
- Apporter de l'eau dans les contenants d'élevage (opération optionnelle selon la variante de l'invention considérée) ;
- Grouper en unité élémentaire d'élevage les contenants d'élevage (opération optionnelle selon la variante de l'invention considérée) ;
- Stocker l'unité élémentaire d'élevage en environnement contrôlé (dans la première zone Z1).

Dans l'exemple représenté, l'ilot D est spécialisé dans le lavage des contenants d'élevage. Il peut en particulier comprendre un ou plusieurs tunnels de lavage D1 adaptés pour le lavage de bacs d'élevage et/ou des palettes.

L'ilot de lavage D est, dans l'exemple ici représenté, configuré pour permettre, lorsque nécessaire, la fourniture de contenants propres à destination des ilots B et C.

Un exemple de séquence de lavage peut mettre en œuvre la séquence des opérations suivantes (certaines opérations pouvant être interverties) :
- Déstocker une unité élémentaire d'élevage (de la première zone Z1);
- Dégrouper partiellement ou totalement les contenants d'élevage d'une unité élémentaire d'élevage ;
- Vider les contenants d'élevage ;
- Séparer les déjections et le substrat des larves vivantes ;
- Nettoyer les contenants d'élevage (cette opération comprenant le lavage et le séchage des contenants) ;
- Stocker les contenants dans une zone dédiée avant réemploi, ou envoyer les contenants pour réemploi ;

Les ilots B et C correspondent dans l'exemple représenté à un premier ilot modulaire B et un second ilot modulaire C. Les ilots B et C sont dits modulaires en ce qu'ils comprennent un certain nombre d'équipements facilement interchangeables ou évolutifs afin de pouvoir être facilement spécialisés pour diverses séquences et/ou opérations d'élevage. Dans la configuration représentée, les ilots modulaires B, C comprennent chacun une zone logistique B1, C1 équipée par exemple d'un de manutention poly-articulé, par exemple un robot six-axes, ou sept axes. Le robot équipant ces zones permet le dégroupement des contenants d'élevage (opération de « dégrouper partiellement ou totalement les contenants d'élevage d'une unité élémentaire d'élevage «) lorsque cela est nécessaire pour la réalisation subséquente d'une opération d'élevage, et optionnellement le groupement et la palettisation des contenants en unités élémentaires (opération de « grouper en unité élémentaire d'élevage les contenants d'élevage ») après réalisation d'une opération d'élevage au niveau de l'ilot correspondant. Le robot de manutention permet également, le cas échéant, de vider les contenants, par exemple dans des trémies alimentant les machines de l'ilot.

L'ilot est également configuré pour permettre la réalisation d'autres opérations sur des unités élémentaires ou des contenants. L'ilot comporte ainsi un ou plusieurs postes, ou une ou plusieurs machines, vers lesquelles les unités élémentaires, les contenants, ou le contenu des contenants, doivent être envoyés. Cette fonction peut être en partie assurée par le robot six-axes, par exemple pour la dépose d'un contenant sur un convoyeur amenant l'unité élémentaire ou le contenant vers un poste donné.

Dans l'exemple représenté, le premier ilot modulaire B comporte un premier séparateur B2, configuré pour la séparation entre larves vivantes, larves mortes, et déjections.

Le premier ilot modulaire B comporte également notamment un deuxième séparateur B3, configuré pour la calibration des larves (vivantes), c'est-à-dire la ségrégation des larves en fonction de leur taille ou de leur masse. De nombreuses machines de tri sont envisageables. Une multitude d'équipements de séparation est connue, qui permettent de trier des insectes selon :
- leur taille ;
- leur stade physiologique ;
- le fait qu'ils soient morts ou vivants ;
et/ou de séparer les déchets fins (déjections, substrat non consommé, mues...) des insectes.

Parmi ces équipements, on peut citer par exemple les tables de séparation, les systèmes de tri optique, les tamis vibrants, etc.

Un équipement peut être configuré et paramétré pour réaliser différents tris.

Le nombre d'équipements de séparation mis en œuvre peut être adapté aux besoins de l'élevage considéré.

Le deuxième séparateur B3 peut ainsi être employé typiquement dans une séquence de calibration des larves selon leur taille, qui peut comporter la séquence des opérations suivantes (certaines opérations pouvant être interverties ou parallélisées), et qui est schématiquement représentée à la figure 4:
- Déstocker (S1) une unité élémentaire d'élevage (de la première zone Z1);
- Dégrouper (S2) partiellement ou totalement les contenants d'élevage d'une unité élémentaire d'élevage ;
- Vider (S3) les contenants d'élevage ;
- Nettoyer (S4) les contenants d'élevage (en parallèle des opérations de séparation et calibration suivantes) ;
- Séparer (S5) les déjections et les larves vivantes ;
- Calibrer (S6) des larves selon leur taille ;
- Remplir (S7) des contenants d'élevage avec les insectes (c'est-à-dire dans ce cas les larves vivantes, et selon la calibration précédente) ;
- Déposer (S8) du substrat d'élevage dans les contenants d'élevage ;
- Grouper (S9) en unité élémentaire d'élevage les contenants d'élevage ;
- Apporter de l'eau dans un contenant d'élevage (opération optionnelle selon la variante de l'invention considérée) ;
- Stocker (S10) les unités élémentaires d'élevage en environnement contrôlé (dans la première zone Z1).

Cette séquence peut typiquement être employée pour la synchronisation des insectes au stade larvaire. Tel que bien représenté à la figure 4, les étapes de « déposer (S8) du substrat » et de « grouper (S9) les contenants » peuvent, selon la variante du procédé considérée, être interverties. De même, les contenants propres employés à l'étape de « remplir (S7) des contenants » ne sont pas nécessairement les mêmes contenants que ceux vidés à l'étape de « vider (S3) ». Les étapes de « séparer (S5) » et « calibrer (S6) » d'une part et de « nettoyer (S4) » d'autre part peuvent être menées en parallèle ou séquentiellement, étant indépendantes.

Dans l'exemple représenté, le second ilot modulaire C comporte un séparateur (par exemple un tamiseur C2), configuré pour la séparation entre insectes adultes, œufs, et substrat d'élevage (milieu ajouté dans les contenants adaptés à la vie des insectes ou larves ou nymphes). Il peut notamment s'agir d'un tamiseur à étage, les tamis successifs séparant les étages étant de plus en plus fins afin de réaliser la séparation précitée. Néanmoins, d'autres types de machines, peuvent être employés, tel que précédemment mentionné. Le second ilot modulaire C comporte dans l'exemple représenté également un troisième séparateur ventilateur C3 (ou autre outil de séparation), configuré pour la séparation entre insectes adultes, larves et nymphes.

Le second ilot modulaire C comporte également un quatrième séparateur ventilateur C4 (ou autre outil de séparation), configuré pour la séparation entre insectes adultes vivants et insectes morts. Ce quatrième séparateur ventilateur C4 (ou autre outil de séparation) peut ainsi être employé typiquement dans une séquence de tri des adultes vivants et des morts, comportant la séquence des opérations suivantes (certaines opérations pouvant être interverties) :
- Déstocker une unité élémentaire d'élevage (de la première zone Z1) ;
- Dégrouper partiellement ou totalement les contenants d'élevage d'une unité élémentaire d'élevage ;
- Vider les contenants d'élevage ;
- Trier les adultes vivants des adultes morts ;
- Remplir des contenants d'élevage avec les insectes (c'est-à-dire dans ce cas les adultes vivants, selon le tri précédent) ;
- Grouper en unité élémentaire d'élevage les contenants d'élevage ;
- Déposer du substrat d'élevage dans un contenant d'élevage ;
- Apporter de l'eau dans un contenant d'élevage (opération optionnelle selon la variante de l'invention considérée) ;
- Stocker les unités élémentaires d'élevage en environnement contrôlé (dans la première zone Z1).

Selon la variante du procédé considérée, les étapes de « déposer du substrat » et de « grouper les contenants » peuvent notamment être interverties.

Dans l'exemple ici représenté, le second ilot modulaire C comporte également un cinquième séparateur ventilateur C5, configuré pour la séparation entre larves et nymphes. Ce séparateur est optionnel.

L'organisation d'atelier, et en particulier de la seconde zone Z2 présentée ici à titre d'exemple, permet la réalisation de l'ensemble des opérations périodiques d'élevage d'insectes, de l'œuf jusqu'à l'obtention d'insectes adultes ayant le niveau de croissance souhaité. De nombreuses autres organisations, sont possibles, mettant en œuvre un nombre plus élevé ou moindre d'ilots ou de postes.

En particulier, afin d'optimiser le rendement de l'élevage, il convient d'élever les insectes (œufs, larves, nymphes, adultes...) avec une densité optimisée dans les contenants. La densité d'insectes peut typiquement correspondre à une masse d'insectes par volume ou par surface de contenant ou un nombre d'insectes par volume ou par surface de contenant.

Une densité optimisée peut typiquement correspondre à la densité d'insectes pour laquelle une phase de stockage s'achève avec une densité considérée maximale garantissant la bonne santé des insectes dans l'unité élémentaire ou les contenants (une éventuelle marge de sécurité étant le cas échéant appliquée). Cela peut être obtenu par la mise en œuvre d'une séquence dite de concentration/dilution. Dans une séquence dite de concentration/dilution des insectes, la densité d'insectes dans chaque contenant d'une unité d'élevage est amenée à une densité cible par ajout d'insectes, retrait d'insectes, ou répartition des insectes dans des contenants. La densité cible correspond à une densité prédéterminée souhaitée au début de la phase de stockage subséquente.

En particulier, la densité cible peut correspondre à une densité pour laquelle il est estimé que l'intervalle temporel souhaité séparant la séquence de concentration/dilution courante d'une séquence de concentration/dilution suivante s'achèvera avec une densité considérée maximale garantissant la bonne santé des insectes dans les contenants. Une façon simple de définir une densité cible est de lui associer un pourcentage prédéfini de la densité considérée maximale d'insectes garantissant la bonne santé des insectes dans les contenants.

Plus simplement, la densité cible peut correspondre à la moitié de la densité atteinte en fin phase de croissance précédente, ce qui conduit par exemple à simplement répartir également les insectes de chaque contenant en deux contenants. De manière analogue, les insectes de chaque contenant peuvent être également répartis en trois, quatre, ou plus de contenants. D'autres répartitions sont possibles, par exemple les insectes de deux contenants étant également répartis dans trois contenants.

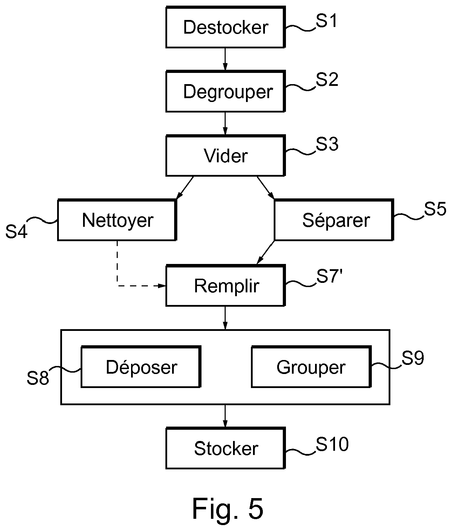

En particulier la séquence opératoire de concentration/dilution, représentée à la figure 5, peut comprendre la séquence des opérations suivantes :
- Déstocker (S1) une unité élémentaire d'élevage (de la première zone Z1) ; puis
- Dégrouper (S2) totalement les contenants d'élevage de l'unité élémentaire d'élevage ; puis
- Vider (S3) les contenants d'élevage ; puis
- Nettoyer (S4) les contenants ; et
- Séparer (S5) les déjections et les larves vivantes ; puis
- Remplir (S7') des contenants d'élevage avec des insectes, à la densité cible ; puis
- Déposer (S8) du substrat d'élevage et ou apporter de l'eau ; et
- Grouper (S9) en unités élémentaires d'élevage les contenants d'élevage ; puis
- Stocker (S10) les unités élémentaires d'élevage en environnement contrôlé.

Tel que bien représenté à la figure 5, les étapes de « déposer (S8) du substrat » et de « grouper (S9) les contenants » peuvent, selon la variante du procédé considérée, être interverties. De même, les contenants propres employés à l'étape de « remplir (S7') des contenants » ne sont pas nécessairement les mêmes contenants que ceux vidés à l'étape de « vider (S3) ». Les étapes de « séparer (S5) »t et « nettoyer (S4) » peuvent être menée en parallèle ou séquentiellement, étant indépendantes.

Un élevage d'insectes peut mettre en œuvre par exemple trois procédés parallèles, à savoir : un procédé dit de production, qui concerne l'évolution d'œufs ou de juvéniles jusqu'à un stade larvaire d'une maturité donnée pouvant correspondre au produit final de l'élevage avant éventuelle transformation, un procédé dit de reproduction, qui concerne l'évolution d'œufs ou de juvéniles jusqu'au stade de jeunes adultes, et un procédé dit de ponte, qui concerne la production d'œufs ou de juvéniles par des insectes adultes.

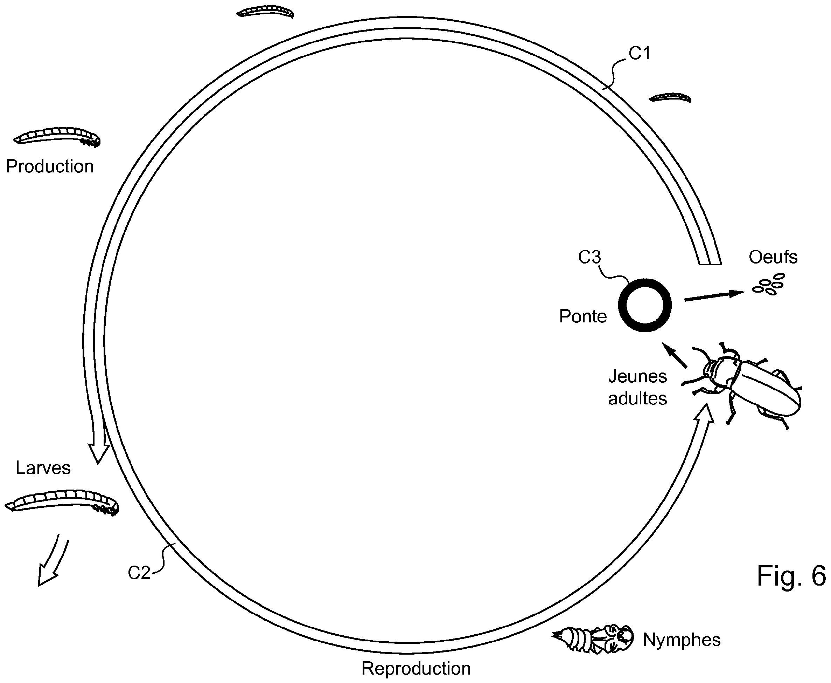

Les trois procédés précités et leur enchaînement chronologique éventuel sont schématiquement représentés à la figure 6, dans l'exemple particulier de l'élevage de ténébrions meuniers dans des conditions environnementales d'élevage optimisées. Les trois procédés sont illustrés de manière cyclique, chacun des procédés pouvant en alimenter un autre ou être alimenté par un autre.

Un arc de cercle C1 correspond au procédé dit de production, qui concerne l'évolution d'œufs ou de juvéniles jusqu'à un stade larvaire d'une maturité donnée pouvant correspondre au produit final de l'élevage avant éventuelle transformation. Ce procédé peut typiquement durer environ 6 à 14 semaines, dans l'exemple représenté.

Un cercle complet C2 correspond au procédé dit de reproduction, qui concerne l'évolution d'œufs ou de juvéniles jusqu'au stade de jeunes adultes, et qui vise en particulier à l'élevage d'insectes destinés à devenir reproducteurs et être intégrés dans un procédé de ponte. Ce procédé peut typiquement durer de 10 à 24 semaines, dans l'exemple représenté.

Un rond C3 représente le procédé dit de ponte, qui concerne la production d'œufs ou de juvéniles par des insectes adultes. Un cycle de ponte d'un adulte donné dure environ une semaine. Les adultes peuvent enchaîner plusieurs cycles de ponte.

Tel que représenté, les adultes destinés à la ponte peuvent être issus du procédé dit de reproduction (que le processus de ponte complète donc pour une partie des adultes obtenus à l'issue dudit processus de reproduction). Les œufs (ou juvéniles) obtenus à l'issue du procédé de ponte sont dirigés soit en entrée d'un procédé de production, soit d'un procédé de reproduction.

Pour chaque procédé, différentes séquences ou opérations d'élevage sont mises en œuvre à des intervalles prédéfinis (correspondant à des phases de croissance), afin d'optimiser l'élevage en termes de croissance, de rendement, de bien-être des insectes.

Typiquement, le procédé de production peut, dans l'exemple représenté, comporter la réalisation de deux séquences de manière assez fréquente, à savoir les séquences de nourrissage (par exemple une à deux fois par semaine) et de nettoyage des bacs (par exemple toutes les deux semaines). Des exemples de successions d'opérations constituant ces séquences ont été préalablement présentés. Le procédé de production peut également comporter, par exemple une à deux fois dans le procédé, la synchronisation des insectes par mise en œuvre d'une séquence de calibration des larves par exemple en employant la succession d'opérations précédemment décrite pour cette séquence. Le procédé de production peut également comporter, par exemple une à deux fois dans le procédé, une séquence de concentration/dilution des insectes selon la succession d'opérations précédemment décrite pour cette séquence.

Le début du procédé de reproduction présente les mêmes séquences d'élevage que le procédé de production. Le procédé de reproduction comporte avantageusement, une fois au cours dudit procédé, une séquence de concentration/dilution menant à une dilution extrême, lors de laquelle la densité cible est très inférieure à la densité initiale d'insectes. Il s'agit en effet, au cours de cette dilution extrême et dans le cadre d'un élevage de ténébrions meuniers ou autres coléoptères, de répartir des contenants comprenant un volume d'insectes (larves, etc.) dans des contenants de sorte à n'obtenir qu'une unique couche d'insectes en fond de contenant.

Le procédé de reproduction comporte également des séquences ou opérations répétées typiquement une fois par semaine, au moins pendant une partie (temporelle) du procédé. Ainsi, les opérations suivantes peuvent être menées de manière assez fréquente :
- Séparer entre eux les larves, les nymphes et les adultes ;
- Séparer les œufs des adultes ;
- Séparer les insectes (nymphes ou adultes) morts des vivants ;
- Séparer les individus malades des individus sains ;
- Sélectionner des insectes destinés au procédé de ponte.

Le procédé de ponte est, lui, la continuation du procédé de reproduction. En effet, les insectes adultes sélectionnés à l'issue du procédé de reproduction sont introduits dans les contenants de ponte, avec des insectes plus âgés déjà actifs en termes de ponte, et après séparation des adultes morts des contenants de ponte. Il s'agit donc d'un procédé circulaire. Le cycle peut être de l'ordre d'une semaine, c'est-à-dire que chaque semaine seront réalisées les opérations de séparation des morts et d'ensemencement. En, outre, périodiquement, le procédé de ponte peut comporter une séquence visant à concentrer les œufs ou les larves (séquence de concentration/dilution avec une densité cible supérieure à la densité initiale).

Le procédé de ponte, en ce qu'il ne comporte pas de séquence de synchronisation, est l'accessoire d'un procédé conforme à l'invention. Par exemple, il peut compléter notamment un procédé de reproduction conforme à une variante de l'invention, venant par exemple lui-même à l'appui d'un procédé de production conforme à une variante de l'invention.

Diverses opérations dites de support peuvent être en outre menées au cours d'un procédé d'élevage. Il peut s'agir notamment :
- de contrôler la bonne santé des insectes via des capteurs (dans les contenants, ou dans la zone de stockage) ou par prise d'image au moment de la réalisation de séquences d'élevage ou d'opérations ;
- de désinfecter les contenants ;
- de réaliser des échantillonnages des lots de production, afin d'analyser finement le contenu des contenants (analyse microbiologique, de la teneur en protéines des insectes, etc.)
- de réaliser des prises de données notamment en vue de garantir la traçabilité du procédé, par exemple réaliser des pesées des contenants tout au long du procédé d'élevage, et alimenter une base de données sur chacun des lots par exemple en y indiquant quelle séquence ou opération a été réalisée et à quel moment, les paramètres employés pour cette séquence (par exemple quelle quantité de nourriture a été apportée) ;
- de garantir un certain brassage génétique en intégrant de nouvelles souches d'insectes périodiquement dans l'élevage.

Le séquençage des opérations d'élevage, bien que simplifié dans l'invention par le fait que les unités élémentaires contiennent des insectes de même stade de développement ou de même maturité, nécessite une organisation et un suivi spatial et temporel des unités élémentaires voire des contenants. Un tel suivi peut être réalisé par dispositif informatique permettant de suivre la réalisation des différentes opérations d'élevage au cours dudit élevage. En particulier, le procédé d'élevage suit une succession d'étapes, c'est-à-dire typiquement un ordonnancement précis des séquences et opérations d'élevage réalisées selon un calendrier prédéfini, qui peut éventuellement être corrigé en cours d'élevage selon l'évolution de la croissance des insectes (œuf, oothèques, larves, pupes, ou nymphes).

Afin de pouvoir suivre de manière efficace l'avancement dans le procédé d'élevage, l'atelier est avantageusement doté d'un système de suivi des unités élémentaires, et/ou de certains contenants, et/ou de chacun des contenants.

Le système de suivi des unités élémentaires, et/ou de certains contenants, et/ou de chacun des contenants peut en particulier mettre en œuvre la technologie RFID (selon l'acronyme anglophone « radio frequency identification » souvent traduit par « radio-identification »). Une étiquette RFID peut être associée le cas échéant aux unités élémentaires, ou contenants, des systèmes de lecture permettant leur identification étant disposés dans l'atelier, typiquement au niveau de l'interface entre la première zone et la seconde zone (pour gérer la position de l'unité élémentaire dans les rayonnages de la première zone Z1), et en entrée et/ou en sortie des différents postes auxquels sont réalisées des séquences ou opérations d'élevage. Le système RFID mis en œuvre peut permettre également l'identification instantanée de l'ensemble des bacs constituant une palette. Ce système RFID est avantageusement lié à une base de données qui permet de garantir la traçabilité de chacun des contenants. La traçabilité porte sur l'ensemble du procédé d'élevage, depuis les matières premières employées pour l'élevage aux insectes (nourriture, substrat, etc.) et jusqu'à l'abattage et les produits finis.

D'autres moyens d'identification et de collecte de données correspondantes peuvent être employés avec succès, par exemple une communication par onde, par exemple selon un protocole Wi-Fi, Bluetooth, ou Zigbee (marques déposées). Un système à bas débit mettant en œuvre des ondes radio basses fréquences peut aussi être mis en œuvre avec succès.

Ainsi, l'atelier est également avantageusement doté d'un système informatique de suivi de production, associé aux moyens de suivi du type RFID ou d'un autre type. La production, et en particulier le procédé d'élevage conforme à l'invention, peut ainsi être pilotée de manière automatisée, le système pouvant typiquement associer certaines séquences ou opérations à certaines unités élémentaires d'élevage, et commander à la fin d'une phase de croissance, la récupération d'une unité élémentaire d'élevage donnée dans la première zone de stockage, la réalisation de la séquence souhaitée, et le retour à une position donnée de l'unité élémentaire.

Typiquement, puisque le stade d'évolution et de croissance des insectes (œufs, larves, nymphes, insectes adultes) dans une même unité élémentaire d'élevage est théoriquement identique, le suivi des unités élémentaires est en général suffisant pour le pilotage des dispositifs automatisés de l'atelier.

Le suivi de certains contenants particuliers peut permettre par exemple le prélèvement périodique et l'échantillonnage de ces contenants particuliers pour la réalisation de contrôles ou de prélèvements.

Enfin, le suivi individuel des contenants, nécessitant l'identification de chacun desdits contenants, permet un suivi complet et individualisé du procédé d'élevage. Il permet notamment de reconstituer au besoin des unités élémentaires en cours d'élevage avec des contenants issus d'autres unités élémentaires ou avec de nouveaux contenants.

Un procédé d'élevage conforme à l'invention peut être employé pour l'élevage de nombreuses espèces d'insectes, moyennant de légères adaptations, typiquement dans la définition et la planification des séquences d'élevage.

Généralement, une seule espèce est élevée dans un atelier. Plusieurs espèces peuvent également être élevées, de préférence dans des parties distinctes de l'atelier. Dans le cadre d'un atelier adapté pour l'élevage simultané de plusieurs espèces d'insectes, certaines synergies peuvent être exploitées. Typiquement, certaines larves, insectes vivants ou morts, d'une espèce, ou les sous-produits de production d'un élevage, peuvent être employées pour nourrir une autre espèce.

Le ou les produit(s) d'intérêt obtenus *in fine,* après valorisation des produits de l'élevage, sont obtenus à partir d'insectes. Tel que précédemment mentionné, on entend par « insectes » des insectes à n'importe quel stade de développement, tel qu'un stade adulte, larvaire ou un stade de nymphe. De préférence, les insectes mis en œuvre dans le procédé selon l'invention sont comestibles.

Plus particulièrement, les insectes peuvent être choisis parmi le groupe constitué par les coléoptères, les diptères, les lépidoptères, les isoptères, les orthoptères, les hyménoptères, les blattoptères, les hémyptères, les hétéroptères, les éphéméroptères et les mécoptères, de préférence, parmi les coléoptères, les diptères, les orthoptères et les lépidoptères.

Préférentiellement, les insectes sont choisis parmi le groupe constitué par *Tenebrio molitor* (ou ténébrion meunier), *Hermetia illucens, Rhynchophorus ferrugineus, Galleria mellonella, Alphitobius diaperinus, Zophobas morio, Blattera fusca, Musca domestica, Chrysomya megacephala, Locusta migratoria, Schistocerca gregaria, Acheta domestica* et *Samia ricini.*

Des conditions favorables notamment dans la première zone où les insectes sont stockés pendant leur croissance peuvent permettre un développement et une reproduction rapide des insectes. Par exemple, le cycle complet de développement du ténébrion meunier, de l'œuf à l'adulte à pleine croissance, peut durer de deux à trois mois à une température de 15°C à 35°C, alors qu'il peut prendre un an dans la nature.

Un procédé conforme à l'invention permet ainsi l'élevage à grande échelle d'insectes avec des coûts minimaux grâce à sa grande optimisation (liée par exemple aux séquences de synchronisation et/ou de concentration/dilution) et une possible grande automatisation.

La croissance des insectes s'effectue dans une zone à atmosphère (température, hygrométrie...) contrôlée voire pilotée, afin de proposer des conditions de croissance optimales pour les insectes à tous les stades de l'œuf à l'adulte. Par la synchronisation et l'éventuelle optimisation de la densité des insectes dans les contenants, le procédé peut présenter un très haut rendement de production. A titre d'exemple, il est estimé qu'en employant dans la première zone Z1 des rayonnages sur 12 mètres de hauteur, un atelier conforme à l'invention pourrait permettre la production de 10000 tonnes par an de farine protéique par hectare employé, alors que la culture de soja permet d'en produire une à cinq tonnes par hectare et par an, et un élevage de porcs ou de poulets en batteries permet de produire un équivalent de quelques dizaines de tonnes par hectare et par an.

Le procédé proposé dans l'invention, fondé sur un ordonnancement séquentiel, dans deux zones distinctes, d'opérations unitaires en alternance avec des périodes « passives » de stockage pour la croissance des insectes, est adapté à la production d'insectes à échelle industrielle. A titre d'exemple, un atelier de taille modeste selon l'invention pourrait produire a minima une tonne de larves par jour, présenter une zone adaptée au stockage de cinquante tonnes d'insectes (œufs, larves, nymphes, et adultes) réparties sur 500 palettes. Les opérations d'élevage nécessitent dans ce cas le déplacement d'environ 100 à 200 palettes par jour. Une exploitation industrielle à grande échelle, pour répondre aux besoins des marchés en alimentation animale par exemple, pourrait ainsi typiquement conduire à l'adoption de valeur cinquante à cent fois supérieures à celles précitées, selon les marchés visés.

Enfin, l'élevage selon un procédé conforme à l'invention peut être réalisé avec des moyens permettant un contrôle et un suivi rigoureux, limitant les risques sanitaires dans l'élevage.

## Revendications

1. Procédé d'élevage d'insectes comportant des phases de croissance lors desquelles les insectes sont stockés dans un environnement contrôlé, lesdites phases de croissance alternant avec des séquences opératoires lors desquelles au moins une opération ponctuelle d'élevage est réalisée,
ladite opération ponctuelle d'élevage étant l'une des opérations suivantes :
• déposer (S8) un substrat d'élevage dans un contenant d'élevage ;
• apporter de l'eau dans un contenant d'élevage ;
• vider (S3) un contenant d'élevage ;
• identifier des insectes présentant des symptômes de maladie pour les séparer d'un contenant ;
• séparer des déchets fins, comprenant des déjections, des restes de substrat et des mues, et les insectes ;
• séparer des larves vivantes matures et le substrat non consommé ;
• séparer des larves mortes de larves vivantes ;
• calibrer (S6) des larves selon leur taille ;
• trier les insectes adultes des larves et des nymphes ;
• trier les insectes vivants des insectes morts ;
• trier des œufs des adultes et trier des œufs du substrat ;
• trier des nymphes des larves ;
• nettoyer (S4) un contenant d'élevage ;
• remplir (S7, S7') un contenant d'élevage avec des insectes ;
• jeter le contenu d'un contenant d'élevage,
**caractérisé en ce qu'**il comprend une séquence dite de synchronisation (figure 4) lors de laquelle un lot d'insectes est trié et réparti en plusieurs catégories de taille ou de maturité dans différents contenants, puis lesdits contenants (31, 32) sont regroupés pour constituer des unités élémentaires (UE) d'élevage comprenant un nombre prédéfini de contenants (31, 32), une unité élémentaire (UE) comprenant uniquement des insectes de même catégorie.

2. Procédé d'élevage selon la revendication 1, dans lequel la séquence de synchronisation (figure 4) est menée plusieurs fois sur chaque insecte au cours dudit procédé.

3. Procédé d'élevage selon la revendication 1 ou la revendication 2, comprenant au moins une séquence dite de concentration/dilution (figure 5) des insectes, dans laquelle la densité d'insectes dans chaque contenant (31, 32) d'une unité d'élevage est amenée à une densité cible par ajout d'insectes, retrait d'insectes, ou répartition des insectes dans des contenants (31, 32), la densité cible correspondant à une densité prédéterminée souhaitée au début de la phase de stockage subséquente.

4. Procédé d'élevage selon la revendication 3, dans laquelle la densité cible correspond à la densité pour laquelle il est estimé que, tenant compte de la croissance des insectes prévue lors de la phase de stockage subséquente, l'intervalle temporel souhaité séparant la séquence de concentration/dilution courante d'une séquence de concentration/dilution suivante s'achèvera avec une densité considérée maximale garantissant la bonne santé des insectes dans les contenants (31, 32).

5. Procédé d'élevage selon la revendication 3, dans laquelle la densité cible est définie par un ratio de la densité dans les contenants (31, 32) avant la séquence de concentration/dilution.

6. Procédé d'élevage selon l'une des revendications précédentes comportant, entre chaque phase de croissance, une étape de convoyage automatisé des unités élémentaires (UE) d'élevage contenant les insectes devant faire l'objet d'au moins une séquence opératoire, depuis une zone de stockage dans laquelle se déroule ladite phase de croissance, vers une zone opératoire comportant les postes de travail adaptés des opérations d'élevage de la séquence opératoire.

7. Procédé d'élevage selon l'une des revendications précédentes, comportant des séquences opératoires de nourrissage et/ou des séquences opératoires d'apport d'eau.

8. Procédé d'élevage selon l'une des revendications précédentes, comportant une séquence opératoire de prélèvement d'insectes adultes pour les destiner à la ponte.

9. Procédé d'élevage selon l'une des revendications précédentes, comportant les séquences opératoires suivantes :
- la récupération des œufs des contenants (31, 32) d'élevage contenant des insectes destinés à la ponte,
- la concentration des œufs dans des contenants (31, 32) d'élevage.

10. Procédé d'élevage selon l'une des revendications précédentes, comportant les séquences opératoires suivantes :
- la récupération des juvéniles des contenants (31, 32) d'élevage contenant des insectes destinés à la ponte,
- la concentration des juvéniles dans des contenants (31, 32) d'élevage.

11. Procédé d'élevage selon l'une des revendications précédentes, comportant une séquence opératoire de vidage et nettoyage des contenants (31, 32) d'élevage en vue de leur réemploi.

12. Procédé d'élevage selon l'une des revendications précédentes, dans lequel les séquences opératoires comportent la succession ordonnée de plusieurs opérations parmi les opérations suivantes :
• Stocker (S10) une unité élémentaire d'élevage en environnement contrôlé ;
• Déstocker (S1) une unité élémentaire d'élevage ;
• Dégrouper (S2) partiellement ou totalement les contenants d'élevage d'une unité élémentaire d'élevage ;
• Grouper (S9) en unité élémentaire d'élevage des contenants d'élevage ;
• Déposer (S8) du substrat d'élevage dans un contenant d'élevage ;
• Apporter de l'eau dans un contenant d'élevage ;
• Vider (S3) un contenant d'élevage ;
• Identifier les individus présentant des symptômes de maladie pour les séparer du contenant ;
• Séparer les déchets fins, comprenant les déjections, les restes de substrat et les mues, des insectes ;
• Séparer les larves vivantes matures et le substrat non consommé ;
• Séparer les larves mortes des larves vivantes ;
• Calibrer (S6) des larves selon leur taille ;
• Trier les insectes adultes des larves et des nymphes ;
• Trier les insectes vivants des insectes morts ;
• Trier les œufs des adultes et trier les œufs du substrat ;
• Trier les nymphes des larves ;
• Nettoyer (S4) un contenant d'élevage ;
• Remplir (S7, S7') un contenant d'élevage avec des insectes ;
• Sortir une unité d'élevage de l'élevage et l'envoyer vers un autre procédé ;
• Faire entrer une nouvelle unité d'élevage ;
• Jeter le contenu d'un contenant d'élevage.

13. Procédé selon la revendication 12 dans laquelle la séquence de synchronisation est réalisée au stade larvaire par mise en œuvre d'une séquence de calibration des larves comportant les opérations suivantes :
• Déstocker (S1) une unité élémentaire d'élevage ;
• Dégrouper (S2) partiellement ou totalement les contenants d'élevage d'une unité élémentaire d'élevage ;
• Vider (S3) les contenants d'élevage ;
• Nettoyer (S4) les contenants d'élevage ;
• Séparer (S5) les déjections et les larves vivantes ;
• Calibrer (S6) des larves selon leur taille ;
• Remplir (S7) des contenants d'élevage avec les larves vivantes ;
• Déposer (S8) du substrat d'élevage dans les contenants d'élevage ;
• Grouper (S9) en unité élémentaire d'élevage les contenants d'élevage ;
• Stocker (S10) les unités élémentaires d'élevage en environnement contrôlé.

14. Procédé selon l'une des revendications 3 à 5 et la revendication 10, dans lequel la séquence opératoire de concentration/dilution comprend les étapes suivantes :
• Déstocker (S1) une unité élémentaire d'élevage ;
• Dégrouper (S2) totalement les contenants d'élevage de l'unité élémentaire d'élevage ;
• Vider (S3) les contenants d'élevage ;
• Nettoyer (S4) les contenants ;
• Séparer (S5) les déjections et les larves vivantes ;
• Remplir (S7') des contenants d'élevage avec des insectes, à la densité souhaitée ;
• Déposer (S8) du substrat d'élevage;
• Grouper (S9) en unités élémentaires d'élevage les contenants d'élevage ;
• Stocker (S10) les unités élémentaires d'élevage en environnement contrôlé.

## Patentansprüche

1. Verfahren zur Insektenaufzucht, umfassend Wachstumsphasen, in denen die Insekten in einer kontrollierten Umgebung gelagert werden, wobei sich die Wachstumsphasen mit Arbeitssequenzen abwechseln, in denen zumindest eine einzelne Aufzuchtmaßnahme erfolgt,
wobei die einzelne Aufzuchtmaßnahme eine der folgenden Maßnahmen ist:
• Ablegen (S8) eines Aufzuchtsubstrats in einen Aufzuchtbehälter;
• Einbringen von Wasser in einen Aufzuchtbehälter;
• Leeren (S3) eines Aufzuchtbehälters;
• Identifizieren von Insekten, die Krankheitssymptome zeigen, um sie von einem Behälter zu trennen;
• Aussondern von Feinabfällen, einschließlich Exkremente, Substratreste und Häutungen, und von Insekten;
• Aussondern von gereiften lebenden Larven und von unbenutztem Substrat;
• Trennen toter Larven von lebenden Larven;
• Kalibrieren (S6) der Larven entsprechend ihrer Größe;
• Aussortieren ausgewachsener Insekten von Larven und Puppen;
• Aussortieren lebender Insekten von toten Insekten;
• Aussortieren von Eiern von ausgewachsenen Tieren und Aussortieren von Eiern aus dem Substrat;
• Aussortieren der Puppen von Larven;
• Reinigen (S4) eines Aufzuchtbehälters;
• Füllen (S7, S7') eines Aufzuchtbehälters mit Insekten;
• Entsorgen des Inhalts eines Aufzuchtbehälters,
**dadurch gekennzeichnet, dass**
es eine sogenannte Synchronisationssequenz (Figur 4) umfasst, während der eine Charge von Insekten sortiert und in mehrere Größen- oder Reifegradekategorien in verschiedenen Behältern unterteilt wird, wonach die Behälter (31, 32) zum Bilden von Aufzuchtgrundeinheiten (UE) gruppiert werden, die eine vordefinierte Anzahl von Behältern (31, 32) umfassen, wobei eine Grundeinheit (UE) nur Insekten der gleichen Kategorie umfasst.

2. Aufzuchtverfahren nach Anspruch 1, wobei im Laufe des Verfahrens die Synchronisationssequenz (Figur 4) mehrmalig an jedem Insekt erfolgt.

3. Aufzuchtverfahren nach Anspruch 1 oder Anspruch 2, umfassend zumindest eine Sequenz, als Insektenkonzentrierungs/-ausdünnungssequenz bezeichnet (Figur 5), bei der die Dichte an Insekten in jedem Behälter (31, 32) einer Aufzuchteinheit durch Hinzufügen von Insekten, Entfernen von Insekten oder Verteilen der Insekten in Behältern (31, 32) auf eine Solldichte gebracht wird, wobei die Solldichte einer vorgegebenen, gewünschten Dichte zu Beginn der nachfolgenden Lagerphase entspricht.

4. Aufzuchtverfahren nach Anspruch 3, wobei die Solldichte der Dichte entspricht, für die unter Berücksichtigung des während der nachfolgenden Lagerphase zu erwartenden Insektenwachstums geschätzt wird, dass das gewünschte Zeitintervall zwischen der aktuellen Konzentrierungs-/Ausdünnungssequenz und einer nachfolgenden Konzentrierungs-/Ausdünnungssequenz mit einer als maximal betrachteten Dichte endet, die den guten Gesundheitszustand der Insekten in den Behältern (31, 32) gewährleistet.

5. Aufzuchtverfahren nach Anspruch 3, wobei die Solldichte durch ein Verhältnis der Dichte in den Behältern (31, 32) vor der Konzentrierungs-/Ausdünnungssequenz definiert ist.

6. Aufzuchtverfahren nach einem der vorangehenden Ansprüche, umfassend zwischen jeder Wachstumsphase einen Schritt des automatisierten Förderns von Aufzuchtgrundeinheiten (UE), die die Insekten enthalten, die zumindest einer Arbeitssequenz ausgesetzt werden sollen, von einem Lagerbereich, in dem die Wachstumsphase stattfindet, zu einem Arbeitsbereich, der angepasste Arbeitsstationen der Aufzuchtmaßnahmen der Arbeitssequenz enthält.

7. Aufzuchtverfahren nach einem der vorangehenden Ansprüche, umfassend Arbeitssequenzen zur Fütterung und/oder Arbeitssequenzen zur Wasserversorgung.

8. Aufzuchtverfahren nach einem der vorangehenden Ansprüche, umfassend eine Arbeitssequenz zum Entnehmen von ausgewachsenen Insekten, die zur Eiablage bestimmt sind.

9. Aufzuchtverfahren nach einem der vorangehenden Ansprüche, umfassend die nachstehenden Arbeitssequenzen:
- Gewinnen von Eiern aus den Aufzuchtbehältern (31, 32), die Insekten enthalten, die zur Eiablage bestimmt sind,
- Konzentrieren der Eier in den Aufzuchtbehältern (31, 32).

10. Aufzuchtverfahren nach einem der vorangehenden Ansprüche, umfassend die nachstehenden Arbeitssequenzen:
- Gewinnen von Jungtieren aus den Aufzuchtbehältern (31, 32), die Insekten enthalten, die zur Eiablage bestimmt sind,
- Konzentrieren von Jungtieren in den Aufzuchtbehältern (31, 32).

11. Aufzuchtverfahren nach einem der vorangehenden Ansprüche, umfassend eine Arbeitssequenz zum Entleeren und Reinigen von Aufzuchtbehältern (31, 32) zu deren Wiederverwendung.

12. Aufzuchtverfahren nach einem der vorangehenden Ansprüche, wobei die Arbeitssequenzen die geordnete Abfolge mehrerer Maßnahmen unter den folgenden Maßnahmen umfassen:
• Einlagern (S10) einer Aufzuchtgrundeinheit in einer kontrollierten Umgebung;
• Auslagern (S1) einer Aufzuchtgrundeinheit;
• teilweises oder vollständiges Degruppieren (S2) der Aufzuchtbehälter einer Aufzuchtgrundeinheit;
• Gruppieren (S9) der Aufzuchtbehälter zu Aufzuchtgrundeinheiten;
• Ablegen (S8) von Aufzuchtsubstrat in einen Aufzuchtbehälter;
• Einbringen von Wasser in einen Aufzuchtbehälter;
• Entleeren (S3) eines Aufzuchtbehälters;
• Identifizieren von Individuen, die Krankheitssymptome zeigen, um sie von dem Behälter zu trennen;
• Aussondern von Feinabfällen, einschließlich Exkremente, Substratreste und Häutungen, von den Insekten;
• Aussondern gereifter lebender Larven und von unbenutztem Substrat;
• Trennen toter Larven von lebenden Larven;
• Kalibrieren (S6) der Larven entsprechend ihrer Größe;
• Aussortieren ausgewachsener Insekten von den Larven und Puppen;
• Aussortieren lebender Insekten von toten Insekten;
• Aussortieren der Eier von den ausgewachsenen Insekten und Aussortieren der Eier von dem Substrat;
• Aussortieren der Puppen von den Larven;
• Reinigen (S4) eines Aufzuchtbehälters;
• Füllen (S7, S7') eines Aufzuchtbehälters mit Insekten;
• Entfernen einer Aufzuchteinheit aus der Aufzucht und Weiterleiten zu einem weiteren Verfahren;
• Einführen einer neuen Aufzuchteinheit;
• Entsorgen des Inhalts eines Aufzuchtbehälters.

13. Verfahren nach Anspruch 12, wobei
die Synchronisationssequenz im Larvenstadium durch Ausführen einer Sequenz zum Kalibrieren der Larven erfolgt, die folgende Maßnahmen umfasst:
• Auslagern (S1) einer Aufzuchtgrundeinheit;
• teilweises oder vollständiges Degruppieren (S2) der Aufzuchtbehälter einer Aufzuchtgrundeinheit;
• Entleeren (S3) der Aufzuchtbehälter;
• Reinigen (S4) der Aufzuchtbehälter;
• Aussondern (S5) der Exkremente und der lebenden Larven;
• Kalibrieren (S6) der Larven entsprechend ihrer Größe;
• Füllen (S7) der Aufzuchtbehälter mit lebenden Larven;
• Ablegen (S8) von Aufzuchtsubstrat in den Aufzuchtbehältern;
• Gruppieren (S9) der Aufzuchtbehälter zu Aufzuchtgrundeinheiten;
• Einlagern (S10) der Aufzuchtgrundeinheiten in kontrollierter Umgebung.

14. Verfahren nach einem der Ansprüche 3 bis 5 und Anspruch 10, wobei die Konzentrierungs-/Ausdünnungsarbeitssequenz die nachstehenden Schritte umfasst:
• Auslagern (S1) einer Aufzuchtgrundeinheit;
• vollständiges Degruppieren (S2) der Aufzuchtbehälter einer Aufzuchtgrundeinheit;
• Entleeren (S3) der Aufzuchtbehälter;
• Reinigen (S4) der Behälter;
• Aussondern (S5) der Exkremente und der lebenden Larven;
• Füllen (S7') der Aufzuchtbehälter mit Insekten mit der gewünschten Dichte;
• Ablegen (S8) von Aufzuchtsubstrat;
• Gruppieren (S9) der Aufzuchtbehälter zu Aufzuchtgrundeinheiten;
• Einlagern (S10) der Aufzuchtgrundeinheiten in kontrollierter Umgebung.

## Claims

1. Process for rearing insects comprising growth phases during which the insects are stored in a controlled environment, said growth phases alternating with operating sequences during which at least one specific rearing operation is performed,
said specific rearing operation being one of the following operations:
• depositing (S8) a rearing substrate in a rearing container;
• providing water in a rearing container;
• emptying (S3) a rearing container;
• identifying insects exhibiting symptoms of disease in order to separate them from a container;
• separating fine waste, comprising droppings, remains of the substrate and molts, from the insects;
• separating mature live larvae and the unconsumed substrate;
• separating dead larvae from live larvae;
• grading (S6) the larvae according to their size;
• sorting the adult insects from the larvae and the nymphs;
• sorting the live insects from the dead insects;
• sorting the eggs from the adults and sorting the eggs from the substrate;
• sorting the nymphs from the larvae;
• cleaning (S4) a rearing container;
• filling (S7, S7') a rearing container with insects;
• discarding the contents of a rearing container,
**characterised in that** it comprises a sequence called synchronisation sequence (figure 4) during which a batch of insects is sorted and distributed into several categories of size or maturity in different containers, then said containers (31, 32) are grouped together to constitute basic rearing units (UE) comprising a predefined number of containers (31, 32), one basic unit (UE) comprising only insects of the same category.

2. Rearing process according to claim 1, in which the synchronisation sequence (figure 4) performed several times on each insect during said process.

3. Rearing process according to claim 1 or claim 2, comprising at least one sequence called concentration/dilution sequence (Figure 5) of the insects, in which the density of insects in each container (31, 32) of a rearing unit is brought to a target density by the addition of insects, the removal of insects, or the distribution of the insects in containers (31, 32), the target density corresponding to a desired predetermined density at the start of the subsequent storage phase.

4. Rearing process according to claim 3, in which the target density corresponds to the density for which it is estimated that, taking account of anticipated insect growth during the subsequent storage phase, the desired time interval separating the current concentration/dilution sequence from a following concentration/dilution sequence will end with a density considered maximal ensuring the good health of the insects in the containers (31, 32).

5. Rearing process according to claim 3, in which the target density is defined by a density ratio in the containers (31, 32) before the concentration/dilution sequence.

6. Rearing process according to one of the preceding claims comprising, between each growth phase, a step of automated conveying of the basic rearing units (UE) containing the insects which are to be the subject of at least one operating sequence from a storage zone in which said growth stage takes place, to an operating zone comprising workstations appropriate for the rearing operations of the operating sequence.

7. Rearing process according to one of the preceding claims, comprising operating sequences of feeding and/or operating sequences of supplying water.

8. Rearing process according to one of the preceding claims, comprising an operating sequence of taking adult insects for laying purposes.

9. Rearing process according to one of the preceding claims, comprising the following operating sequences:
- collecting eggs from the rearing containers (31, 32) containing insects intended for laying,
- concentrating the eggs in rearing containers (31, 32).

10. Rearing process according to one of the preceding claims, comprising the following operating sequences:
- collecting juveniles from the rearing containers (31, 32) containing insects intended for laying,
- concentrating the juveniles in rearing containers (31, 32).

11. Rearing process according to one of the preceding claims, comprising an operating sequence of emptying and cleaning the rearing containers (31, 32) for the purpose of reusing them.

12. Rearing process according to one of the preceding claims, in which the operating sequences comprise an ordered succession of several operations from among the following operations:
• storing (S10) a basic rearing unit in a controlled environment;
• removing (S1) a basic rearing unit from storage;
• partially or completely ungrouping (S2) the rearing containers of a basic rearing unit;
• grouping (S9) rearing containers to form a basic rearing unit;
• depositing (S8) rearing substrate in a rearing container;
• providing water in a rearing container;
• emptying (S3) a rearing container;
• identifying the individuals exhibiting symptoms of disease in order to remove them from the container;
• separating the fine waste, comprising droppings, the remaining substrate and the molts, from the insects;
• separating the mature live larvae from the unconsumed substrate;
• separating the dead larvae from the live larvae;
• grading (S6) the larvae according to their size;
• sorting the adult insects from the larvae and the nymphs;
• sorting the live insects from the dead insects;
• sorting the eggs from the adults and sorting the eggs from the substrate;
• sorting the nymphs from the larvae;
• cleaning (S4) a rearing container;
• filling (S7, S7') a rearing container with insects;
• removing a rearing unit from rearing and sending it to another process;
• introducing a new rearing unit;
• discarding the contents of a rearing container.

13. Process according to claim 12 in which the synchronisation sequence is performed at the larval stage by carrying out a larva grading sequence comprising the following operations:
• removing (S1) a basic rearing unit from storage;
• partially or completely ungrouping (S2) the rearing containers of a basic rearing unit;
• emptying (S3) the rearing containers;
• cleaning (S4) the rearing containers;
• separating (S5) the droppings from the live larvae;
• grading (S6) the larvae according to their size;
• filling (S7) rearing containers with the live larvae;
• depositing (S8) rearing substrate in the rearing containers;
• grouping together (S9) the rearing containers to form basic rearing units;
• storing (S10) the basic rearing units in a controlled environment.

14. Process according to one of claims 3 to 5 and claim 10, in which the concentration/dilution operating sequence comprises the following steps:
• removing (S1) a basic rearing unit from storage;
• completely ungrouping (S2) the rearing containers of a basic rearing unit;
• emptying (S3) the rearing containers;
• cleaning (S4) the containers;
• separating (S5) the droppings from the live larvae;
• filling (S7') rearing containers with insects at the desired density;
• depositing (S8) rearing substrate;
• grouping (S9) rearing containers to form basic rearing units;
• storing (S10) the basic rearing units in a controlled environment.
